# EUROPÄISCHE PATENTSCHRIFT

(11) **EP 1 097 270 B1**
(45) Veröffentlichungstag und Bekanntmachung des Hinweises auf die Patenterteilung: **04.06.2003**
(21) Anmeldenummer: 99932849.5
(22) Anmeldetag: 07.07.1999
(51) Int. Cl.: D21H 17/72, D06M 13/17, D06M 13/144, D06M 13/224, A61K 7/00, D21H 17/14

(54) **VERWENDUNG VON PIT-EMULSIONEN**
USE OF PIT EMULSIONS
UTILISATION D'EMULSIONS PIT

(30) Priorität: 16.07.1998 GB 9815514; 15.12.1998 GB 9827616
(43) Veröffentlichungstag der Anmeldung: 09.05.2001
(73) Patentinhaber: Cognis Deutschland GmbH & Co. KG, 40589 Düsseldorf (DE)
(72) Erfinder: WADLE, Armin, D-40699 Erkrath (DE); TESMANN, Holger, D-41363 Jüchen (DE); BAUMÖLLER, Guido, D-42799 Leichlingen (DE); LEONARD, Mark, Bexley, Kent DA5 1AZ (GB); ROBBE-TOMINE, Laurence, F-77330 Ozoir-la-Ferrière (FR); ANSMANN, Achim, D-40699 Erkrath (DE); WATCHER, Rolf, D-40595 Düsseldorf (DE); HÖRNER, Viola, D-40593 Düsseldorf (DE); GRIESBACH, Ute, D-40597 Düsseldorf (DE)
(86) Internationale Anmeldenummer: EP9904780
(87) Internationale Veröffentlichungsnummer: WO00004230

(56) Entgegenhaltungen:
- WO-A-95/16824
- WO-A-95/35411
- DE-A- 19 602 902
- GB-A- 2 069 333
- US-A- 3 896 807
- US-A- 4 112 167
- US-A- 4 786 367
- US-A- 5 240 562

## Beschreibung

### Gebiet der Erfindung

Die Erfindung befindet sich auf dem Gebiet der Gebrauchs- und Hygienepapiertücher und betrifft die Verwendung von Emulsionen verschiedener Fettstoffe, die nach dem Phaseninversionstemperaturverfahren hergestellt werden, als lmprägnier- und Avivagemittel.

### Stand der Technik

Unter dem Oberbegriff "Papier" werden ca. 3000 verschiedene Sorten und Artikel verstanden, die sich in ihren Anwendungsgebieten und ihrer Beschaffenheit zum Teil erheblich unterscheiden können. Zu ihrer Herstellung werden eine Reihe von Zusatzstoffen benötigt, von denen Füllstoffe (z.B. Kreide oder Kaolin) und Bindemittel (z.B. Stärke) zu den wichtigsten zählen. Für den Bereich der Tissue- und Hygienepapiere, die in engeren Kontakt mit der menschlichen Haut gebracht werden, besteht ein besonderes Bedürfnis nach einem angenehmen Weichgriff, der dem Papier üblicherweise durch eine sorgfältige Auswahl der Faserstoffe und insbesondere einen hohen Anteil an frischem Holzschliff oder Cellulose verliehen wird. Im Hinblick auf die Wirtschaftlichkeit der Papierherstellung sowie aus ökologischer Sicht, ist es jedoch wünschenswert, möglichst hohe Anteile an qualitativ minderwertigerem deinktem Altpapier mitzuverwenden. Dies hat jedoch zur Folge, daß der Weichgriff des Papiers signifikant verschlechtert wird, was in der Anwendung störend ist und insbesondere bei häufigem Gebrauch auch zu Hautirritationen führen kann.

In der Vergangenheit hat es daher nicht an Versuchen gemangelt, Tissuepapiere durch Tränken, Beschichten oder andere Oberflächenbehandlung so zu behandeln, daß ein angenehmerer Weichgriff resultiert. Gegenstand der internationalen Patentanmeldung **WO 95/35411** (Procter & Gamble) sind Tissuepapiere, die mit Avivagemitteln beschichtet werden, welche 20 bis 80 Gew.-% eines wasserfreien Emulgators (Mineralöle, Fettsäureester, Fettalkoholethoxylate, Fettsäureethoxylate, Fettalkohole und deren Mischungen), 5 bis 95 Gew.-% eines Trägers (Fettalkohole, Fettsäuren oder Fettalkoholethoxylate mit jeweils 12 bis 22 Kohlenstoffatomen im Fettrest) sowie 1 bis 50 Gew.-% Tenside mit einem HLB-Wert von vorzugsweise 4 bis 20 enthalten. Die in der Schrift aufgeführten Ausführungsbeispiele enthalten als Emulgator ausnahmslos Petrolatum. Die internationale Patentanmeldung **WO 95/35412** offenbart ähnliche Tissuepapiere, wobei als Softener wasserfreie Mischungen von (a) Mineralölen, (b) Fettalkoholen oder Fettsäuren und (c) Fettalkoholethoxylaten zum Einsatz kommen. Gegenstand der intenationalen Patentanmeldung **WO 95/16824** (Procter & Gamble) sind Avivagemittel für Tissuepapiere, die Mineralöl, Fettalkoholethoxylate und nichtionische Tenside (Sorbitanester, Glucamide). Des weiteren werden in der internationalen Patentanmeldung **WO 97/30216** (Kaysersberg) Avivagemittel für Papiertaschentücher beschrieben, die (a) 35 bis 90 Gew.-% langkettige Fettalkohole, (b) 1 bis 50 Gew.-% Wachsester mit 24 bis 48 Kohlenstoffatomen, (c) 0 bis 20 Gew.-% nichtionische Emulgatoren und (d) 0 bis 50 Gew.-% Mineralöl enthalten. Vom anwendungstechnischen Standpunkt sind aber Weichgriff und Sensorik der behandelten Papiere nach wie vor verbesserungswürdig.

Die Aufgabe der Erfindung hat somit darin bestanden, Zubereitungen zur Verfügung zu stellen, mit deren Hilfe man Gebrauchspapiere, insbesondere Tissuepapiere, aber auch Tissuegewebe mit besonders angenehmen Weichgriff und ausgezeichneten pflegenden Eigenschaften auch unter Verwendung von solchen Rohstoffen herstellen kann, die einen hohen Altpapieranteil aufweisen. Gleichzeitig sollten nur leicht biologisch abbaubare Hilfsstoffe Verwendung finden und die Zubereitungen leicht in das Tissue eindringen, sich homogen verteilen und auch in hochkonzentrierter Form eine so niedrige Viskosität aufweisen, daß sie sich leicht verarbeiten lassen. Darüber hinaus besteht das Bedürfnis nach Zubereitungen, mit deren Hilfe man Gebrauchspapiere, insbesondere Tissuepapiere, aber auch Tissuegewebe mit besonders hautverträglichen Eigenschaften herstellen kann. Es ist weiterhin wünschenswert die Gebrauchspapiere mit zusätzlichen Eigenschaften, wie beispielsweise entzündungshemmenden oder antimikrobiellen Eigenschaften zu versehen. Darüber hinaus wäre es wünschenswert, Gebrauchspapiere zu erhalten, die ihre pflegenden Eigenschaften über eine längeren Zeitraum behalten. Dies ist insbesondere bei Produkten, die eine längere Lagerung überstehen müssen wünschenswert.

### Beschreibung der Erfindung

Gegenstand der Erfindung ist die Verwendung von PlT-Emulsionen, enthaltend
(a) C₈-C₂₂-, vorzugsweise C₁₂-C₁₈-Fettsäurealkylester,
(b) C_{8-C22-}, vorzugsweise C₁₂₋₁₈₋Fettalkohole,
(c) C₈-C₂₂-, vorzugsweise C₁₂-C₁₈-Alkoholpolyglycolether und
(d) C₈-C₂₂-, vorzugsweise C₁₂-C₁₈ -Fettsäurepartialglyceride
als Imprägnier- und Avivagemittel für Papiere, Vliese und Gewebe, vorzugsweise zur Hautbehandlung.

Überraschenderweise wurde gefunden, daß Zubereitungen der genannten Art geeignet sind, selbst besonders kritischem Tissuepapier mit einem Anteil von bis zu 95 Gew.-% Altpapier sowie auch Tissuegewebe einen angenehmen Weichgriff zu verleihen. Die nach dem Phaseninversionstemperatur-Verfahren hergestellten Emulsionen sind auch hochkonzentriert niedrigviskos, so daß sie sich leicht verarbeiten lassen. Infolge der geringen Tröpfchengröße (< 100 µm) dringen die Emulsionen sehr rasch in die Tissues ein und verteilen sich homogen. Ein weiterer Vorteil besteht ferner darin, daß die praktisch geruchsfreien Zubereitungen ökotoxikologisch unbedenklich sind und insbesondere leicht biologisch abgebaut werden können.

### Tissuepapiere und Tissuegewebe

Tissupapiere, auf die sich die vorliegende Erfindung bezieht, können ein- oder mehrlagig aufgebaut sein. In der Regel weisen die Papiere ein Quadratmetergewicht von 10 bis 65, vorzugsweise 15 bis 30 g und eine Dichte von 0,6 g/cm³ und weniger auf. Beispiele für Tissuepapiere, auf sich die erfindungsgemäße Verwendung erstrecken kann, sind Toilettenpapiere, Papiertaschentücher, Gesichtsreinigungstücher, Abschminktücher, Erfrischungstücher, Haushaltstücher und dergleichen. Je nach Anwendung können die Tücher besondere Wirkstoffe enthalten, beispielsweise Feuchtigkeitsspender, Insektenrepellents (After-Sun-Tücher), Dihydroxyaceton, Deowirkstoffe, Tenside, Alkohole (Erfrischungstücher), pflegende Öle, antiinflammatorische Wirkstoffe (Babytücher) und dergleichen. Neben den papierbasierten Tissues kommen auch entsprechende Tissuegewebe in Frage, die aus Faser- oder Fleecestoff hergestellt werden.

### Fettsäurealkylester

Als Komponente (a) der Avivagemittel kommen Fettsäureaikylester der Formel **(I)** in Frage,

**R**^{**1**}**CO-OR**^{**2**} (I)

in der R¹CO für einen linearen oder verzweigten, gesättigten oder ungesättigten Acylrest mit 8 bis 22, vorzugsweise 12 bis 18 und insbesondere 14 bis 16 Kohlenstoffatomen und R² für einen linearen oder verzweigten Alkyl- und/oder Alkenylrest mit 6 bis 22 Kohlenstoffatomen steht. Typische Beispiele sind die Ester der Caprylsäure, Isononansäure, Caprinsäure, Laurinsäure, Myristinsäure, Palmitinsäure, Palmoleinsäure, Stearinsäure, Isostearinsäure, Ölsäure, Elaidinsäure, Petroselinsäure, Elaeostearinsäure, Arachinsäure, Gadoleinsäure, Behensäure und Erucasäure sowie deren technische Mischungen mit Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, lsostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie wiederum deren technische Mischungen. Vorzugsweise werden Wachsester eingesetzt, also Fettsäurealkylester, die bei 20°C eine plastische, aber feste Konsistenz aufweisen und in Summe 24 bis 48 Kohlenstoffatome aufweisen. Typische Beispiele sind Myristylmyristat, Cetearylisononanoat, Cetylpalmitat, Cetylstearat, Stearylpalmitat, Stearylstearat und dergleichen.

### Fettalkohole

Unter Fettalkoholen, die als Komponente (b) eingesetzt werden können, sind primäre Alkohole zu verstehen, die vorzugsweise der Formel **(II)** folgen,

**R**^{**3**}**OH** (II)

in der R³ für einen linearen oder verzweigten Alkyl- und/oder Alkenylrest mit 8 bis 22, vorzugsweise 12 bis 18 und insbesondere 14 bis 16 Kohlenstoffatomen steht. Typische Beispiele sind Caprylalkohol, Caprinalkohol, Laurylalkohol, Myristylalkohol, Cetylalkohol, Palmoleylalkohol, Stearylalkohol, lsostearylalkohol, Oleylalkohol, Elaidylalkohol, Petroselinylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestem auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkem- oder Talgfettalkohol. Des weiteren kommen auch Guerbetalkohole in Frage, also in 2-Stellung verzweigte primäre Alkohole in Betracht, die man durch basenkatalysierte Kondensation von Fettalkoholen mit 8 bis 10 Kohlenstoffen herstellen kann. Vorzugsweise werden Cetylalkohol, Stearylalkohol, Cetearylalkohol, Behenylalkohol sowie deren Gemische oder 2-Octyldodecanol eingesetzt.

### Alkoholpolyglycolether

Unter Alkoholpolyglycolethern, die die Komponente (c) bilden, sind die Anlagerungsprodukte von Ethylen- und/oder Propylenoxid an Fettalkohole der Gruppe (b) oder Oxoalkohole gleicher Kettenlänge zu verstehen, die vorzugsweise der Formel **(III)** folgen,

**R**^{**4**}**O(CH**_{**2**}**CHR**^{**5**}**O)**_{**n**}**H** (III)

in der R⁴ für einen linearen oder verzweigten Alkyl- und/oder Alkenylrest mit 8 bis 22, vorzugsweise 12 bis 18 Kohlenstoffatomen und insbesondere 14 bis 16, R⁵ für Wasserstoff oder Methyl und n für Zahlen von 1 bis 50 steht. Typische Beispiele sind die Addukte von durchschnittlich 1 bis 50, vorzugsweise 5 bis 40 und insbesondere 10 bis 20 Mol Ethylenoxid an Caprylalkohol, Caprinalkohol, Laurylalkohol, Myristylalkohol, Cetylalkohol, Palmoleylalkohol, Stearylalkohol, lsostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind Addukte von 10 bis 20 Mol Ethylenoxid an technische Fettalkohole mit 16 bis 18 Kohlenstoffatomen, wie beispielsweise Cetea-rylalkohol oder Talgfettalkohol.

### Partialglyceride

Als Komponente (d) enthalten die Mischungen Partialglyceride, die der Formel **(IV)** folgen,

**HOCH**_{**2**}**CH(OH)CH**_{**2**}**OCOR**^{**6**} (IV)

in der R⁶CO für einen linearen oder verzweigten, gesättigten oder ungesättigten Acylrest mit 8 bis 22, vorzugsweise 12 bis 18 und insbesondere 14 bis 16 Kohlenstoffatomen steht. Die Partialglyceride, also Monoglyceride, Diglyceride und deren technische Gemische können herstellungsbedingt noch geringe Mengen Triglyceride enthalten. Typische Beispiele sind Mono- und/oder Diglyceride auf Basis von Caprylsäure, Caprinsäure, Laurinsäure, Palmitinsäure, Palmoleinsäure, Stearinsäure, lsostearinsäure, Ölsäure, Elaidinsäure, Petroselinsäure, Elaeostearinsäure, Arachinsäure, Gadoleinsäure, Behensäure und Erucasäure sowie deren technische Mischungen. Vorzugsweise werden technische Palmitinsäureglyceride, Stearinsäureglyceride, lsostearinsäureglyceride, und/oder Behensäureglyceride eingesetzt, welche einen Monoglyceridanteil im Bereich von 50 bis 95, vorzugsweise 60 bis 90 Gew.-% aufweisen.

### PIT-Emulsionen

In einer bevorzugten Ausführungsform der Erfindung werden PIT-Emulsionen als Imprägnier- und Avivagemittel eingesetzt, die - bezogen auf den Aktivsubstanzgehalt - 30 bis 70 Gew.-% Ölkörper und 70 bis 30 Gew.-% Emulgatoren enthalten. In einer besonders bevorzugten Ausführungsform enthalten die Emulsionen - wiederum bezogen auf den Aktivsubstanzgehalt -
(a) 2 bis 70, vorzugsweise 30 bis 50 Gew.-% C₈-C₂₂-Fettsäurealkylester,
(b) 1 bis 40, vorzugsweise 10 bis 20 Gew.-% C₈-C₂₂-Fettalkohole,
(c) 10 bis 40, vorzugsweise 20 bis 30 Gew.-% C₈-C₂₂-Alkoholpolyglycolether,
(d) 1 bis 40, vorzugsweise 10 bis 20 Gew.-% C₈-C₂₂-Fettsäurepartialglyceride und
(e) 0 bis 70, vorzugsweise 10 bis 50 Gew.-% Hilfs- und Zusatzstoffe,
mit der Maßgabe, daß sich die Mengenangaben zu 100 Gew.-% ergänzen. Der Aktivsubstanzgehalt der Emulsionen kann je nach Anwendungszweck zwischen 0,5 und 80 Gew.-% liegen. Bei höheren Aktivsubstanzgehalten nimmt die Fließfähigkeit der Emulsionen stark ab, bei niedrigeren Gehalten ist der anwendungstechnische Effekt nicht mehr festzustellen. Vorzugsweise werden Konzentrate mit einem Aktivsubstanzgehalt im Bereich von 10 bis 70 Gew.-% in den Handel gebracht, die dann auf eine Anwendungskonzentration von 1 bis 15 Gew.-% verdünnt werden. Die wäßrige Phase kann dabei falls gewünscht auch Polyole, vorzugsweise bis zu 15 Gew.-% Glycerin enthalten.

### Pflegende Öle

In einer weiteren bevorzugten Ausführungsform der Erfindung werden als Hilfs- und Zusatzstoffe, die die Komponente (e) bilden, pflegende Öle eingesetzt. Als pflegende Öle kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe in Betracht. Vorzugsweise beträgt die Einsatzmenge der pflegenden Öle - bezogen auf den Aktivsubstanzgehalt der PIT-Emulsionen - 10 bis 50 Gew.-%. Sind pflegende Öle in diesen angegebenen Mengen in den Emulsionen enthalten, reduziert sich der Anteil der Komponente (a) auf vorzugsweise 2 bis 30 Gew.-%. Die technische Lehre besteht also darin, bei Bedarf eine Teilmenge der Esteröle, die die Komponente (a) ausmachen, gegen pflegende Öle auszutauschen.

### Co-Emulgatoren

Falls gewünscht, können die erfindungsgemäß zu verwendenden Zubereitungen weitere Emulgatoren, vorzugsweise nichtionische, kationische oder amphotere Emulgatoren enthalten, als da sind:
(1) C_{12/18}-Fettsäuremono- und -diester von Anlagerungsprodukten von 1 bis 30 Mol Ethylenoxid an Glycerin;
(2) Glycerinmono/diester, Sorbitanmono/diester und Zuckermono/diester von gesättigten und ungesättigten Fettsäuren mit 6 bis 22 Kohlenstoffatomen oder Hydroxycarbonsäuren mit 2 bis 6 Kohlenstoffatomen, wie z.B. Citronensäure, Äpfelsäure oder Weinsäure, und deren Ethylenoxidanlagerungsprodukte;
(3) Alkylmono- und -oligoglycoside mit 8 bis 22 Kohlenstoffatomen im Alkylrest und deren ethoxylierte Analoga;
(4) Anlagerungsprodukte von 15 bis 60 Mol Ethylenoxid an Ricinusöl und/oder gehärtetes Ricinusöl;
(5) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(6) Anlagerungsprodukte von 2 bis 15 Mol Ethylenoxid an Ricinusöl und/oder gehärtetes Ricinusöl;
(7) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(8) Mono-, Di- und Trialkylphosphate sowie Mono-, Di- und/oder Tri-PEG-alkylphosphate und deren Salze;
(9) Wollwachsalkohole;
(10) Polysiloxan-Polyalkyl-Polyether-Copolymere bzw. entsprechende Derivate;
(11) Mischester aus Pentaerythrit, Fettsäuren, Citronensäure und Fettalkohol gemäß DE-PS 1165574 und/oder Mischester von Fettsäuren mit 6 bis 22 Kohlenstoffatomen, Methylglucose und Polyolen, vorzugsweise Glycerin oder Polyglycerin sowie
(12) Polyalkylenglycole.

Die Anlagerungsprodukte von Ethylenoxid und/oder von Propylenoxid an Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C₁₂/₁₈-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N, N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C₈/₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpmpionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind, da sie den Weichgriff weiter verbessem. Unter der Bezeichnung "Esterquats" werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Aus der Deutschen Patentschrift **DE-C1 4308794** (Henkel) ist überdies ein Verfahren zur Herstellung fester Esterquats bekannt, bei dem man die Quatemierung von Triethanolaminestem in Gegenwart von geeigneten Dispergatoren, vorzugs-weise Fettalkoholen, durchführt. Übersichten zu diesem Thema sind beispielsweise von R.Puchta et al. in **Tens.Surf.Det, 30, 186 (1993)**, M.Brock in **Tens.Surf.Det. 30, 394 (1993)**, R.Lagerman et al. in **J.Am.Oil.Chem.Soc., 71, 97 (1994)** sowie l.Shapiro in **Cosm.Toil. 109, 77 (1994)** erschienen. Die quatemierten Fettsäuretriethanolaminestersalze folgen der Formel **(V)**, 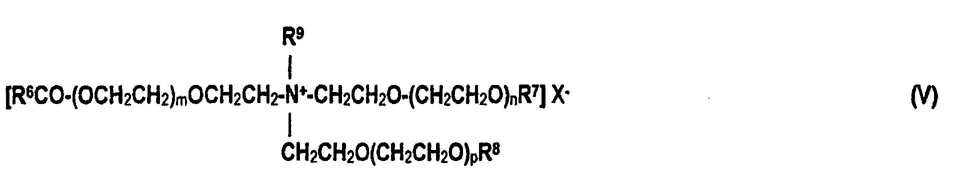 in der R⁶CO für einen Acylrest mit 6 bis 22 Kohlenstoffatomen, R⁷ und R⁸ unabhängig voneinander für Wasserstoff oder R⁶CO, R⁹ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, lsostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}- Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2:1 bis 2,2:1, vorzugsweise 1,5:1 bis 1,9:1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich quatemierte Fettsäuretriethanolaminestersalze der Formel **(V)** als besonders vorteilhaft erwiesen, in der R⁶CO für einen Acylrest mit 16 bis 18 Kohlenstoffatomen, R⁷ für R⁶CO, R⁸ für Wasserstoff, R⁹ für eine Methylgruppe, m, n und p für 0 und X für Methylsulfat steht. Neben den quatemierten Fettsäuretriethanolaminestersalzen kommen als Esterquats femer auch quaternierte Estersalze von Fettsäuren mit Diethanolalkylaminen der Formel **(VI)** in Betracht, 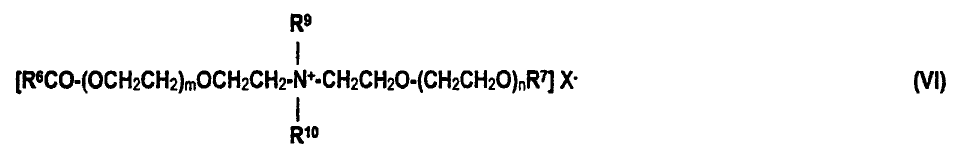 in der R⁶CO für einen Acylrest mit 6 bis 22 Kohlenstoffatomen, R⁷ für Wasserstoff oder R⁶CO, R⁹ und R¹⁰ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als weitere Gruppe geeigneter Esterquats sind schließlich die quaternierten Estersalze von Fettsäuren mit 1,2-Dihydroxypropyldialkylaminen der Formel **(VII)** zu nennen, 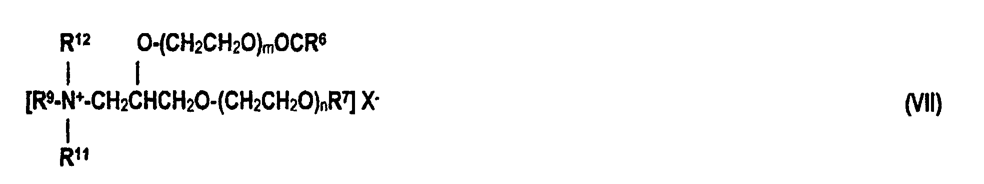 in der R⁶CO für einen Acylrest mit 6 bis 22 Kohlenstoffatomen, R⁷ für Wasserstoff oder R⁶CO, R⁹, R¹⁰ und R¹¹ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Hinsichtlich der Auswahl der bevorzugten Fettsäuren und des optimalen Veresterungsgrades gelten die für **(V)** genannten Beispiele auch für die Esterquats der Formeln **(VI)** und **(VII)**.

In einer weiteren Ausführungsform der Erfindung enthalten die PIT Emulsionen als Hilfs- und Zusatzstoffe Wirkstoffe.

### Wirkstoffe - Chitosane

In einer besonderen Ausführungsform der Erfindung enthalten die PlT-Emulsionen als Wirkstoffe (e-1) Chitosane. Überraschenderweise wurde festgestellt, daß durch Zusatz von Chitosanen zu den PIT-Emulsionen nicht nur Gebrauchspapiere mit einem besonderen Weichgriff sondern auch mit erheblich verbesserter Hautverträglichkeit erhalten werden. Hierbei wird insbesondere durch die Filmbildungseigenschaften der Chitosane ein Moisturizingeffekt erzielt und das Hautgefühl der Emulsion besonders in der Langzeitwirkung verbessert. Des weiteren wird die Haftung von weiteren Wirkstoffen, wie beispielsweise UV-Filtern oder Parfümölen erhöht Zusätzlich wirken die Chitosane antimikrobiell und verleihen somit den mit diesen Emulsionen ausgerüsteten Gebrauchspapieren diese Eigenschaft.

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

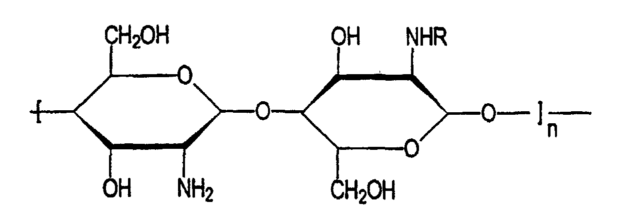

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-232**). Übersichten zu diesem Thema sind auch beispielsweise von B. Gesslein et al. in **HAPPI 27, 57 (1990)**, O. Skaugrud in **Drug Cosm.Ind. 148,24 (1991)** und E. Onsoyen et al. in **Seifen-Öle-Fette-Wachse 117, 633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol. Chem. 177 3589 (1976)** oder der französischen Patentanmeldung **FR 2701266 A** bekannt. Vorzugsweise werden solche Typen eingesetzt, wie sie in den deutschen Patentanmeldungen **DE 4442987 A1** und **DE 19537001 A1** (Henkel) offenbart werden und die ein durchschnittliches Molekulargewicht von 800.000 bis 1.200.000 Dalton, eine Viskosität nach Brookfield (1 gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Neben den Chitosanen als typischen kationischen Biopolymeren kommen im Sinne der Erfindung auch anionisch bzw. nichtionisch derivatisierte Chitosane, wie z.B. Carboxylierungs-, Succinylierungs- oder Alkoxylierungsprodukte in Frage, wie sie beispielsweise in der deutschen Patentschrift **DE 3713099 C2** (L'Oreal) sowie der deutschen Patentanmeldung **DE 19604180 A1** (Henkel) beschrieben werden. In einer bevorzugten Ausführungsform werden Chitosane mit einem durchschnittlichen Molekulargewicht von 10.000 bis 5.000.000 Dalton, insbesondere von 30.000 bis 100.000 Dalton sowie insbesondere 800.000 bis 1.200.000 eingesetzt.. Die Chitosane können in den erfindungsgemäßen Mitteln in Mengen von 0,001 bis 2,5, vorzugsweise 0,01 bis 0,5 Gew.-% - bezogen auf den Aktivsubstanzgehalt - enthalten sein.

### Wirkstoffe -(Desoxy)Ribonucleinsäuren

In einer weiteren bevorzugten Ausführungsform enthalten die PIT-Emulsionen als Wirkstoffe (e-2) (Desoxy)Ribonucleinsäuren. Überraschenderweise wurde festgestellt, daß die mit den so zusammengesetzten PIT-Emulsionen behandelten Gebrauchspapiere besondere entzündungshemmende und antioxidative Eigenschaften aufweisen. In einer weiteren Ausführungsform der Erfindung enthalten die PIT-Emulsionen Chitosane (e-1) und (Desoxy)Ribonucleinsäuren (e-2). Überraschenderweise wurde festgestellt, daß durch Kombination von Chitosanen und (Desoxy)Ribonucleinsäuren PIT-Emulsionen erhalten werden, die den Gebrauchspapieren überragende pflegende Eigenschaften sowie gleichzeitig entzündungshemmende Eigenschaften verleihen. Hier bei ist insbesondere zu erwähnen, daß diese Eigenschaften langanhaltend sind.

Unter (Desoxy)Ribonucleinsäuren (DNA bzw. RNA) werden hochmolekulare, fadenförmige Polynucleotide verstanden, die sich von 2'-Desoxy-ß-D-ribonucleosiden bzw. D-Ribonucleosiden ableiten, die ihrerseits wieder von äquivalenten Mengen einer Nucleobase und der Pentose 2-Desoxy-D-ribo-furanose bzw. D-Ribofuranose aufgebaut werden. Der Einsatz von Nucleinsäuren als Wirkstoffe in der Kosmetik ist bekannt. So werden beispielsweise in der französischen Patentanmeldung **FR-A1 2511253** Hautund Sonnenschutzmittel mit einem Gehalt an hochpolymerisierter DNA vorgeschlagen. Aus der japanischen Offenlegungsschrift **JP-A2 62/096404** (Kanebo) sind kosmetische Zusammensetzungen mit Nucleinsäuren und Diisopropylamindichloracetat bekannt. Gegenstand der franzö-sischen Patentschrift **FR-B1 2620024** (Soc.d'Etudes Dermatologiques) sind Zubereitungen, enthaltend Nucleinsäurederivate als Radikalfänger. Beispiele sind Adenin, Guanosin, Xanthin, Hypoxanthin, Uracil und Ribonucleinsäure. In der internationalen Patentanmeldung **WO 95/01773** (Boston University) wird ein Verfahren zur Stimulation der Pigmentproduktion beschrieben, bei dem man DNA-Fragmente, bevorzugt Dinucleotide, in liposomaler Form in die Epidermis transportiert. Gegenstand der deutschen Patentanmeldung **DE-A1 4323615** sind schließlich Zusammensetzungen mit einem Gehalt an Nucleinsäuren und deren Fragmenten als Anti-Ageing- und Sonnenschutzcremes.

Als Nucleobasen können die DNA bzw. RNA die Purinderivate Adenin und Guanin sowie die Pyrimidine Cytosin und Thymin bzw. Uracil enthalten. In den Nucleinsäuren sind die Nucleobasen N-glykosidisch mit Kohlenstoffatom 1 der Ribose, wodurch im Einzelfall Adenosine, Guanosine, Cytidine und Thimidine entstehen. In den Säuren verknüpft eine Phosphatgruppe die 5'-Hydroxygruppe der Nucleoside mit der 3'-OH-Gruppe der jeweils folgenden durch eine Phosphodiesterbrücke unter Ausbildung von Einzelstrang-DNA bzw. -RNA. In der Regel liegt die DNA doppelsträngig vor (Ausbildung von Wasserstoffbrückenbindungen zwischen den entsprechenden Basen) und die RNA einzelsträngig. Je nach Behandlung der Nucleinsäuren, können sowohl DNA als auch RNA in Form von Doppelsträngen und/oder Einzelsträngen vorliegen. Auch Heterodimere (Doppelstrang zwischen DNA und RNA) sind möglich. Unter dem Begriff (Desoxy)Ribonucleinsäuren im Sinne der vorliegenden Erfindung werden sowohl doppel- als auch einzelsträngige (Desoxy)Ribonucleinsäuren verstanden, des weiteren Mischungen aus Einzelsträngen und Doppelsträngen. Wegen des großen Verhältnisses von Länge zu Durchmesser neigen DNA- bzw. RNA-Moleküle schon bei mechanischer Beanspruchung, etwa bei der Extraktion, zu Strangbruch. Aus diesem Grunde kann das Molekulargewicht der Nucleinsäuren 10³ bis 10⁹ Dalton erreichen. Im Sinne der Erfindung werden konzentrierte DNA bzw. RNA-Lösungen eingesetzt, die sich durch ein flüssig-kristallines Verhalten auszeichnen. Vorzugsweise werden Desoxy- bzw. Ribonucleinsäuren eingesetzt, die aus marinen Quellen beispielsweise durch Extraktion von Fischsperma erhalten werden und die ein Molekulargewicht im Bereich von 1000 bis 5.000.000 Dalton aufweisen. Besonders bevorzugt ist der Einsatz von einzelsträngigen Desoxyribonucleinsäuren marinen Ursprungs mit einem Molekulargewicht im Bereich von 10.000 bis 100.000 Dalton. Die (Desoxy)Ribonucleinsäuren können in den erfindungsgemäßen Mitteln in Mengen von 0,001 bis 2,5, vorzugsweise 0,01 bis 0,5 Gew.-% - bezogen auf den Aktivsubstanzgehalt - enthalten sein.

In einer besonders bevorzugten Ausführungsform enthalten die Emulsionen - wiederum bezogen auf den Aktivsubstanzgehalt -
(a) 2 bis 70, vorzugsweise 30 bis 50 Gew.-% C₈-C₂₂-Fettsäurealkylester,
(b) 1 bis 40, vorzugsweise 10 bis 20 Gew.-% C₈-C₂₂-Fettalkohole,
(c) 10 bis 40, vorzugsweise 20 bis 30 Gew.-% C₈-C₂₂-Alkoholpolyglycolether,
(d) 1 bis 40, vorzugsweise 10 bis 20 Gew.-% C₈-C₂₂Fettsäurepartialglyceride und
(e) 0 bis 70, vorzugsweise 10 bis 50 Gew.-% Hilfs- und Zusatzstoffe, wobei diese
   (e-1) 0,001 bis 2,5, vorzugsweise 0,01 bis 0,5 Gew.-% Chitosane und gegebenenfalls
   (e-2) 0 bis 2,5, vorzugsweise 0 bis 0,5 Gew.-% (Desoxy)Ribonucleinsäuren enthalten,
mit der Maßgabe, daß sich die Mengenangaben zu 100 Gew.-% ergänzen.

In einer weiteren bevorzugten Ausführungsform enthalten die Emulsionen - wiederum bezogen auf den Aktivsubstanzgehalt -
(a) 2 bis 70, vorzugsweise 30 bis 50 Gew.-% C₈-C₂₂-Fettsäurealkylester,
(b) 1 bis 40, vorzugsweise 10 bis 20 Gew.-% C₈-C₂₂-Fettalkohole,
(c) 10 bis 40, vorzugsweise 20 bis 30 Gew.-% C₈-C₂₂-Alkoholpolyglycolether,
(f) 1 bis 40, vorzugsweise 10 bis 20 Gew.-% C₈-C₂₂-Fettsäurepartialglyceride und
(g) 0 bis 70, vorzugsweise 10 bis 50 Gew.-% Hilfs- und Zusatzstoffe, wobei diese
   (e-1) 0,001 bis 2,5, vorzugsweise 0,01 bis 0,5 Gew.-% (Desoxy)Ribonucleinsäuren und gegebenenfalls
   (e-2) 0 bis 2,5, vorzugsweise 0 bis 0,5 Gew.-% Chitosane enthalten,
   mit der Maßgabe, daß sich die Mengenangaben zu 100 Gew.-% ergänzen.

### Weitere Wirkstoffe

In einer weiteren Ausführungsform der Erfindung enthalten die PIT-Emulsionen Wirkstoffe, wie beispielsweise milde Tenside, pflegende Öle, Überfettungsmittel, Stabilisatoren, Konsistenzgeber, Verdickungsmittel, Polymere, Siliconverbindungen, biogene Wirkstoffe, Deowirkstoffe, Antischuppenmittel, Antiaknemittel, Filmbildner, Konservierungsmittel, Hydrotrope, Solubilisatoren, Antioxidantien, Insektenrepellentien, Selbstbräuner, Parfümöle, UV-Filter, Farbstoffe und dergleichen.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Überfettungsmittel** können Substanzen wie beispielsweise Lecithin, Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethyl-cellulose und Hydroxyethylcellulose, ferner höhermolekulare Polyethylenglycolmono- und - diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quatemierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quaternierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacryiat-Copolymere, Vinylacetat/Butylmaleat/Isobomylacrytat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden. Unter **biogenen Wirkstoffen** sind beispielsweise Tocopherol, Tocopherolacetat, Tocopherolpalmitat, Ascorbinsäure, Carotine, Desoxyribonucleinsäure, Retinol, Bisabolol, Allantoin, Phytantriol, Panthenol, AHA-Säuren, Aminosäuren, Ceramide, Pseudoceramide, essentielle Öle, proteolytische Enzyme und Vitaminkomplexe zu verstehen.

Als **Deowirkstoffe** kommen z.B. Antiperspirantien wie etwa Aluminiumchlorhydate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eiweiß- und/oder Polysaccharidfällung [vgl. **J.Soc.Cosm.Chem. 24,281 (1973)**]. Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26,531 (1975)**]. Neben den Chlorhydraten können auch Aluminiumhydroxylactate sowie saure Aluminium/Zirko-niumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan und Chlorhexidingluconat. Besonders wirkungsvoll hat sich auch 5-Chlor-2-(2,4-dichlorphenoxy)-phenol erwiesen, das unter der Marke Irgasan®von der Ciba-Geigy, Basel/CH vertrieben wird.

Als **Antischuppenmittel** können Climbazol, Octopirox und Zinkpyrethion eingesetzt werden. Gebräuchliche **Filmbildner** sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108.95 (1993)** entnommen werden

Weiterhin können die PlT-Emulsionen **Antioxidantien** enthalten. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Camosin und deren Derivate (z.B. Anserin), Carotinoide, Carotine (z.B. α-Carotin, β-Garotin, Lycopin) und deren Derivate, Chlorogensäure und deren Derivate, Liponsäure und deren Derivate (z.B. Dihydroliponsäure), Auro-thioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester ) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können femer Hydrotrope, wie beispielsweise Ethanol, lsopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Formaldehydlösung, Parabene, Pentandiol oder Sorbinsäure sowie die in Anlage 6, Teil A und B der Kosmetikverordnung aufgeführten weiteren Stoffklassen. Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättem (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutem und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxy-ethylisobutyrat, p-tert.-Butylcyclohexylacetat, Linalylacetat, Dimethylbenzylcarbinylacetat, Phenylethyl-acetat, Linalylbenzoat, Benzylformiat, Ethylmethyl-phenylglycinat, Allylcyclohexylpropionat, Styrallylpro-pionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, ∝-Iso-methylionon und Methyl-cedrylketon, zu den Alkoholen Anethol, Citronellol, Eugenol, Isoeugenol, Gera-niol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwen-det, die gemeinsam eine ansprechende Duftnote erzeugen. Auch ätherische öle geringerer Flüch-tigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiver-öl, Olibanöl, Galbanumöl, Labolanumöl und Lavandinöl. Vorzugsweise werden Bergamotteöl, Dihydro-myrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damas-cone, Geraniumöl Bourbon, Cyclohexylsalicylat, Vertofix Coeur, lso-E-Super, Fixolide NP, Evemyl, Iraldein gamma, Phenylessigsäure, Geranylacetat, Benzylacetat, Rosenoxid, Romilllat, lrotyl und Floramat allein oder in Mischungen, eingesetzt.

Unter **UV-Lichtschutzfaktoren** sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP 0693471 B1** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon, wie in der **EP 0818450 A1** beschrieben oder Dioctyl Butamido Triazone (Uvasorb® HEB);
- Propan-1,3-dione, wie z.B. 1-(4-tert Butylphenyl)-3-(4'methoxyphenyl)propan-1,3-dion;
- Ketotricyclo(5.2.1.0)decan-Derivate, wie in der **EP 0694521 B1** beschrieben.

Als wasserlösliche Substanzen kommen in Frage:
- 2-Phenylbenzimidazol-5-sulfonsäure und deren Alkali-, Erdalkali-, Ammonium-, Alkyiammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D.L-Camosin, D-Camosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl- γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind Diese Farbstoffe werden üblicherweise in Konzentrationen von 0,001 bis 0,1 Gew.-%, bezogen auf die gesamte Mischung, eingesetzt.

### Behandlung der Tissuepapiere mit den Avivagemitteln

Die Behandlung der Tissuepapiere mit den Avivagemitteln kann in an sich bekannter Weise erfolgen, wobei die Lösung mindestens auf eine Seite der Papiere aufgetragen wird. Hierzu eignen sich grundsätzlich alle einschlägig bekannten Methoden, mit deren Hilfe man Flüssigkeiten oder Schmelzen auf mehr oder weniger feste Oberflächen auftragen kann, wie z.B. Versprühen, Drucken (z.B. Flexodruck), Beschichten (Gravurbeschichtung), Extrusion sowie Kombinationen dieser Verfahren. Es ist ebenso möglich, die Tücher mit den Zubereitungen zu tränken. Nach dem Auftragen der Zubereitungen schließt sich in der Regel ein kurzer Trockenschritt an. Ausführlich werden Verfahren zum Behandeln von Tissuepapieren mit Avivagemitteln in der schon eingangs genannten Schriften WO 95/35411 und WO 97/30216 beschrieben, auf die hiermit ausdrücklich Bezug genommen wird.

### Beispiele

Zur Prüfung der anwendungstechnischen Eigenschaften wurden handelsübliche dreilagige Tissuepapiere mit einem Anteil an Recyclepapier von 95 % und einem Gewicht von 18 g/m² mit den erfindungsgemäßen PIT-Emulsionen 1 bis 10 sowie den Vergleichszubereitungen V1, V2, V3 und V4 (Non-PlT) in Mengen von jeweils 2,5 g/m² behandelt. Die Papiere wurden anschließend 30 min bei 30°C getrocknet und der Weichgriff dann von einem Panel bestehend aus 6 erfahrenen Testern auf einer Skala von (+++) sehr weich bis (+) weich beurteilt. Des weiteren wurde das sensorische Gefühl beim Betasten der Tücher beurteilt. Die Ergebnisse, die Mittelwerte von drei Versuchsreihen darstellen, sind in Tabellen 1a und 1b wiedergegeben.

**Tabelle 1a**

| **Weichgriff von Tissuepapieren unter Verwendung von Emulsionen** | | | | | | | |
|---|---|---|---|---|---|---|---|
| **Zusammensetzung / Performance** | **1** | **2** | **3** | **4** | **5** | **V1** | **V2** |
| Myristyl Myristate | - | - | - | 15,0 | - | - | 20,0 |
| Cetyl Palmitate | 10,0 | - | - | 1,0 | 12,0 | - | - |
| Cetearyl lsononanoate | - | 15,0 | - | - | - | - | - |
| Isostearyl lsononanoate | - | - | 20,0 | - | 3,0 | - | - |
| Cetearyl Alcohol | 15,0 | 8,0 | 8,0 | 4,0 | 6,0 | 35,0 | 20,0 |
| Ceteareth-12 | 2,0 | 1,0 | 1,0 | 0,5 | - | 15,0 | - |
| Ceteareth-20 | 8,0 | 7,0 | 7,0 | 2,0 | 8,0 | - | 20,0 |
| Glyceryl Palmitate | - | 5,0 | 5,0 | - | 3,0 | - | - |
| Glyceryl Stearate | 5,0 | - | - | 5,0 | - | - | - |
| Dipalmitoylethyl Hydroxyethyl-monium Methosulfate | - | 1,0 | - | 1,0 | - | - | - |
| Octyldodecanol | - | - | - | 8,0 | 3,0 | 15,0 | - |
| Calendula Oil | - | - | - | 5,0 | - | - | - |
| Aleo Vera | - | - | - | - | 10,0 | 1,0 | - |
| Glycerol | 3,0 | 3,0 | 2,5 | - | - | - | - |
| Water | ad 100 | | | | | | |
| ***Weichgriff*** | *++* | *+++* | *++* | +++ | ++ | + | *+* |
| ***Sensorische Beurteilung*** | feucht | feucht | feucht | trocken | trocken | stumpf | Stumpf |

**Tabelle 1b**

| **Weichgriff von Tissuepapieren unter Verwendung von Emulsionen** | | | | | | | |
|---|---|---|---|---|---|---|---|
| **Zusammensetzung / Performance** | **6** | **7** | **8** | **9** | **10** | **V3** | **V4** |
| Myristyl Myristate | - | - | 15,0 | 15,0 | 15,0 | - | 15,0 |
| Cetyl Palmitate | 10,0 | 10,0 | 1,0 | 1,0 | 1,0 | 10,0 | 1,0 |
| Cetearyl Isononanoate | - | - | - | - | - | - | - |
| Isostearyl Isononanoate | - | - | - | - - | | | - |
| Cetearyl Alcohol | 15,0 | 15,0 | 4,0 | 4,0 | 4,0 | 15,0 | 4,0 |
| Ceteareth-12 | 2,0 | 2,0 | 0,5 | 0,5 | 0,5 | 2,0 | 0,5 |
| Ceteareth-20 | 8,0 | 8,0 | 2,0 | 2,0 | 2,0 | 8,0 | 2,0 |
| Glyceryl Palmitate | - | - | - | - | - | - | - |
| Glyceryl Stearate | 5,0 | 5,0 | 5,0 | 5,0 | 5,0 | 5,0 | 5,0 |
| Dipalmitoylethyl Hydroxyethyl-monium Methosulfate | - | - | 1,0 | 1,0 | 1,0 | - | 1,0 |
| Octyldodecanol | - | - | 8,0 | 8,0 | 8,0 | - | 8,0 |
| Calendula Oil | - | - | 5,0 | 5,0 | 5,0 | - | 5,0 |
| Chitosan ¹⁾ | 0,025 | 0,1 | 0,05 | 0,1 | - | - | - |
| Desoxyribonucleinsäure²⁾ | - | 0,1 | - | 0,1 | 0,1 | - | - |
| Glycerol | 3,0 | 3,0 | - | - | - | 3,0 | - |
| Water | ad 100 | | | | | | |
| Weichgriff | ++ | +++ | ++ | +++ | ++ | + | + |
| Sensorische Beurteilung | feucht | feucht | Trok-ken | trocken | trocken | stumpf | stumpf |

| | | | | | | | |
|---|---|---|---|---|---|---|---|
| 1) Hydagen HCMF, Henkel KGaA | | | | | | | |
| 2) Desoxyribonucleinsäure: Molekulargewicht ca. 70000, Reinheit (bestimmt durch spektro-photometrische Messung der Absorption bei 260 nm sowie 280 nm): mindestens 1,7. | | | | | | | |

In der nachfolgenden Tabellen 2a und 2b sind eine Reihe von Formulierungsbeispielen angegeben. Dabei bedeuten (11-13 und 23-25) Formulierungen für Baby-Reinigungstücher, (14,15,26,27) O/W-Emulsionen für Softcremetücher, (16-21, 28-33) Körperpflegeemulsionen und (22,34) After-Sün-Emulsionen.

**Tabelle 2a**

| **Formuiierungsbeisplele** | | | | | | |
|---|---|---|---|---|---|---|
| **Zusammensetzung** | **11** | **12** | **13** | **14** | **15** | **16** |
| Glyceryl Stearate (and) Ceteareth-20 (and) Ceteareth-12 (and) Cetearyl Alcohol (and) Cetyl Palmitate* | 9,5 | 13,5 | 11,4 | 8,5 | 13,1 | 10,4 |
| Ceteareth-20 | 2,4 | 0,4 | 1,1 | 2,9 | 0,4 | 2,9 |
| Cocoglycerides | 5,0 | - | 10,0 | - | 6,0 | - |
| C12/15 Benzoate | - | 5,0 | - | 4,0 | - | - |
| Dicaprylyl Carbonate | - | 6,0 | 6,0 | 4,0 | - | 5,0 |
| Dacaprylyl Ether | 5,0 | - | - | - | 4,0 | - |
| Dimethicone | 3,0 | 6,0 | - | - | 4,0 | |
| Dioctyl Malate | - | - | - | 8,0 | - | - |
| Cetearyl lsononanoate | 6,0 | - | - | 5,0 | - | - |
| Oleyl Erucate | - | 3,0 | - | - | 4,0 | 4,0 |
| Almond Oil | - | - | 2,0 | - | - | - |
| Octyldodecanol | - | 2,0 | - | - | - | 8,0 |
| Hexyldecanol (and) Hexyldecyl Laurate | - | - | - | 3,0 | 5,0 | - |
| Panthenol | 1,0 | | | | | |
| Bisabolol | 0,2 | | | | | |
| Tocopherol / Tocopherol Acetate | 1,0 | | | | | |
| Glycerin | 3,0 | | | | | |
| Wasser | ad 100 | | | | | |

| | | | | | | |
|---|---|---|---|---|---|---|
| *) Emulgade® SE, Henkel KGaA | | | | | | |

**Tabelle 2a**

| **Formulierungsbeispiele (Forts.)** | | | | | | |
|---|---|---|---|---|---|---|
| **Zusammensetzung** | **17** | **18** | **19** | **20** | **21** | **22** |
| Glyceryl Stearate (and) Ceteareth-20 (and) Ceteareth-12 (and) Cetearyl Alcohol (and) Cetyl Palmitate* | 12,4 | 9,2 | 8,3 | 14,0 | 13,5 | 15,5 |
| Ceteareth-20 | 0,5 | 2,4 | 3,1 | 0,2 | 0,3 | - |
| Cocoglycerides | 5,0 | - | - | - | 5,0 | 4,0 |
| C12/15 Benzoate | - | 4,0 | - | - | - | - |
| Dicaprylyl Carbonate | - | - | - | - | - | 3,0 |
| Dacaprylyl Ether | 6,0 | - | 5,0 | 8,0 | - | - |
| Dimethicone | 3,0 | 6,0 | - | 2,0 | 4,0 | 2,0 |
| Cyclomethicone | - | - | 3,0 | - | - | - |
| Cetearyl lsononanoate | - | 6,0 | - | - | 3,0 | 3,0 |
| Oleyl Erucate | - | 3,0 | 4,0 | - | 5,0 | - |
| Almond Oil | 4,0 | - | 1,0 | - | 4,0 | - |
| Octyldodecanol | - | - | - | 10,0 | - | 8,0 |
| Hexyldecanol (and) Hexyldecyl Laurate | - | - | 6,0 | - | - | - |
| Panthenol | 1,0 | | | | | |
| Bisabolol | 0,2 | | | | | |
| Tocopherol / Tocopherol Acetate | 1,0 | | | | | |
| Glycerin | 3,0 | | | | | |
| Wasser | ad 100 | | | | | |

| | | | | | | |
|---|---|---|---|---|---|---|
| *) Emulgade® SE, Henkel KGaA | | | | | | |

**Tabelle 2b**

| **Formulierungsbeispiele** | | | | | | |
|---|---|---|---|---|---|---|
| **Zusammensetzung** | **23** | **24** | **25** | **26** | **27** | **28** |
| Glyceryl Stearate (and) Ceteareth-20 (and) Ceteareth-12 (and) Cetearyl Alcohol (and) Cetyl Paimitate^{*} | 9,5 | 13,5 | 11,4 | 8,5 | 13,1 | 10,4 |
| Ceteareth-20 | 2,4 | 0,4 | 1,1 | 2,9 | 0,4 | 2,9 |
| Cocoglycerides | 5,0 | - | 10,0 | - | 6,0 | - |
| C12/15 Benzoate | - | 5,0 | - | 4,0 | - | - |
| Dicaprylyl Carbonate | - | 6,0 | 6,0 | 4,0 | - | 5,0 |
| Dacaprylyl Ether | 5,0 | - | - | - | 4,0 | - |
| Dimethicone | 3,0 | 6,0 | - | - | 4,0 | - |
| Dioctyl Malate | - | - | - | 8,0 | - | - |
| Cetearyl Isononanoate | 6,0 | - | - | 5,0 | - | - |
| Oleyl Erucate | - | 3,0 | - | - | 4,0 | 4,0 |
| Almond Oil | - | - | 2,0 | - | - | - |
| Octyldodecanol | - | 2,0 | - | - | - | 8,0 |
| Chitosan ¹⁾ | 0,1 | 0,1 | 0,1 | 0,15 | 0,15 | 0,08 |
| Desoxyribonucleinsäure ²⁾ | 0,1 | 0,1 | 0,1 | 0,1 | 0,1 | 0,1 |
| Hexyldecanol (and) Hexyldecyl Laurate | - | - | - | 3,0 | 5,0 | - |
| | | | | | | |
| Panthenol | 1,0 | | | | | |
| Bisabolol | 0,2 | | | | | |
| Tocopherol / Tocopherol Acetate | 1,0 | | | | | |
| Glycerin | 3,0 | | | | | |
| Wasser | ad 100 | | | | | |

| | | | | | | |
|---|---|---|---|---|---|---|
| *) Emulgade® SE, Henkel KGaA | | | | | | |
| 1) Hydagen HCMF, Henkel KGaA | | | | | | |
| 2) Desoxyribonucleinsäure: Molekulargewicht ca. 70000, Reinheit (bestimmt durch spektro-photometrische Messung der Absorption bei 260 nm sowie 280 nm): mindestens 1,7. | | | | | | |

**Tabelle 2b**

| **Formulierungsbeispiele (Forts.)** | | | | | | |
|---|---|---|---|---|---|---|
| **Zusammensetzung** | **29** | **30** | **31** | **32** | **33** | **34** |
| Glyceryl Stearate (and) Ceteareth-20 (and) Ceteareth-12 (and) Cetearyl Alcohol (and) Cetyl Palmitate* | 12,4 | 9,2 | 8,3 | 14,0 | 13,5 | 15,5 |
| Ceteareth-20 | 0,5 | 2,4 | 3,1 | 0,2 | 0,3 | - |
| Cocoglycerides | 5,0 | - | - | - | 5,0 | 4,0 |
| C12/15 Benzoate | - | 4,0 | - | - | - | - |
| Dicaprylyl Carbonate | - | - | - | - | - | 3,0 |
| Dacaprylyl Ether | 6,0 | - | 5,0 | 8,0 | - | - |
| Dimethicone | 3,0 | 6,0 | - | 2,0 | 4,0 | 2,0 |
| Cyclomethicone | - | - | 3,0 | - | - | - |
| Cetearyl lsononanoate | - | 6,0 | - | - | 3,0 | 3,0 |
| Oleyl Erucate | - | 3,0 | 4,0 | - | 5,0 | - |
| Almond Oil | 4,0 | - | 1,0 | - | 4,0 | - |
| Octyldodecanol | - | - | - | 10,0 | - | 8,0 |
| Chitosan ¹⁾ | 0,08 | 0,08 | 0,1 | 0,05 | 0,05 | 0,1 |
| Desoxyribonucleinsäure ²⁾ | 0,1 | 0,1 | 0,1 | 0,1 | 0,1 | 0,1 |
| Hexyldecanol (and) Hexyldecyl Laurate | - | - | 6,0 | - | - | - |
| Panthenol | 1,0 | | | | | |
| Bisabolol | 0,2 | | | | | |
| Tocopherol / Tocopherol Acetate | 1,0 | | | | | |
| Glycerin | 3,0 | | | | | |
| Wasser | ad 100 | | | | | |

| | | | | | | |
|---|---|---|---|---|---|---|
| *) Emulgade® SE, Henkel KGaA | | | | | | |
| 1)Hydagen HCMF, Henkel KGaA | | | | | | |
| 2) Desoxyribonucleinsäure: Molekulargewicht ca. 70000, Reinheit (bestimmt durch spektro-photometrische Messung der Absorption bei 260 nm sowie 280 nm): mindestens 1,7. | | | | | | |

## Patentansprüche

1. Verwendung von PIT-Emulsionen, enthaltend
(a) C₈-C₂₂-Fellsäurealkylester,
(b) C₈-C₂₂-Fettalkohole,
(c) C₈-C₂₂-Alkoholpolyglycolether und
(d) C₈-C₂₂-Fettsäurepartialglyceride
als lmprägnier- und Avivagemittel für Papiere, Vliese und Gewebe.

2. Verwendung nach Anspruch 1, **dadurch gekennzeichnet, daß** man als Komponente (a) Fettsäurealkylester der Formel **(I)** einsetzt,
**R**^{**1**}**CO-OR**^{**2**} (I)
in der R¹CO für einen linearen oder verzweigten, gesättigten oder ungesättigten Acylrest mit 8 bis 22 Kohlenstoffatomen und R² für einen linearen oder verzweigten Alkyl- und/oder Alkenylrest mit 6 bis 22 Kohlenstoffatomen steht.

3. Verwendung nach den Ansprüchen 2 und/oder 3, **dadurch gekennzeichnet, daß** man als Komponente (b) Fettalkohole der Formel **(II)** einsetzt,
**R**^{**3**}**OH** (II)
in der R³ für einen linearen oder verzweigten Alkyl- und/oder Alkenylrest mit 8 bis 22 Kohlenstoffatomen steht.

4. Verwendung nach mindestens einem der Ansprüche 1 bis 3, **dadurch gekennzeichnet, daß** man als Komponente (c) Alkoholpolyglycolether der Formel **(III)** einsetzt,
**R**^{**4**}**O(CH**_{**2**}**CHR**^{**5**}**O)**_{**n**}**H** (III)
in der R⁴ für einen linearen oder verzweigten Alkyl- und/oder Alkenylrest mit 8 bis 22 Kohlenstoffatomen, R⁵ für Wasserstoff oder Methyl und n für Zahlen von 1 bis 50 steht.

5. Verwendung nach mindestens einem der Ansprüche 1 bis 4, **dadurch gekennzeichnet, daß** man als Komponente (d) Partialglyceride der Formel **(IV)** einsetzt,
**HOCH**_{**2**}**CH(OH)CH**_{**2**}**OCOR**^{**6**} (IV)
in der R⁶CO für einen linearen oder verzweigten, gesättigten oder ungesättigten Acylrest mit 8 bis 22 Kohlenstoffatomen steht.

6. Verwendung nach mindestens einem der Ansprüche 1 bis 5, **dadurch gekennzeichnet, daß** man PIT-Emulsionen einsetzt, enthaltend - bezogen auf den Aktivsubstanzgehalt -
(a) 2 bis 70 Gew.-% C₈-C₂₂-Fettsäurealkylester,
(b) 1 bis 40 Gew.-% C₈-C₂₂-Fettalkohole,
(c) 10 bis 40 Gew.-% C₈-C₂₂-Alkoholpolyglycolether,
(d) 1 bis 40 Gew.-% C₈-C₂₂-Fettsäurepartialglyceride und gegebenenfalls
(e) 0 bis 70 Gew.-% Hilfs- und Zusatzstoffe mit der Maßgabe, daß sich die Mengenangaben zu 100 Gew.-% ergänzen.

7. Verwendung nach mindestens einem der Ansprüche 1 bis 6, **dadurch gekennzeichnet, daß** man PIT-Emulsionen mit einem Aktivsubstanzgehalt im Bereich von 0,5 bis 80 Gew.-% einsetzt.

8. Verwendung nach mindestens einem der Ansprüche 1 bis 7, **dadurch gekennzeichnet, daß** man PIT-Emulsionen einsetzt, die als Hilfs- und Zusatzstoffe pflegende Öle enthalten.

9. Verwendung nach mindestens einem der Ansprüche 1 bis 8, **dadurch gekennzeichnet, daß** man PIT-Emulsionen einsetzt, die als Hilfs- und Zusatzstoffe nichtionische, amphotere und/oder kationische Co-Emulgatoren enthalten.

10. Verwendung nach mindestens einem der Ansprüche 1 bis 9, **dadurch gekennzeichnet, daß** man PIT-Emulsionen einsetzt, die als Hilfs- und Zusatzstoffe Wirkstoffe enthalten.

11. Verwendung nach mindestens einem der Ansprüche 1 bis 10, **dadurch gekennzeichnet, daß** man PlT-Emulsionen einsetzt, die als Wirkstoffe
(e-1) Chitosane und/oder
(e-2) (Desoxy)Ribonucleinsäuren enthalten

12. Verwendung nach mindestens einem der Ansprüche 1 bis 11, **dadurch gekennzeichnet, daß** man als Komponente (e-1) Chitosane einsetzt mit einem durchschnittlichen Molekulargewicht von 10.000 bis 5.000.000 Dalton.

13. Verwendung nach mindestens einem der Ansprüche 1 bis 12, **dadurch gekennzeichnet, daß** man als Komponente (e-1) Chitosane einsetzt mit einem durchschnittlichen Molekulargewicht von 30.000 bis 100.000 Dalton.

14. Verwendung nach mindestens einem der Ansprüche 1 bis 13, **dadurch gekennzeichnet, daß** man als Komponente (e-1) Chitosane einsetzt mit einem durchschnittlichen Molekulargewicht von 800.000 bis 1.200.000 Dalton.

15. Verwendung nach mindestens einem der Ansprüche 1 bis 14, **dadurch gekennzeichnet, daß** man als Komponente (e-1) Chitosane einsetzt, die ausgewählt sind aus der Gruppe bestehend aus anionisch derivatisierten Chitosanen, nichtionisch derivatisierten Chitosanen und kationisch derivatisierten Chitosanen.

16. Verwendung nach mindestens einem der Ansprüche 1 bis 15, **dadurch gekennzeichnet, daß** man PlT-Emulsionen einsetzt, enthaltend - bezogen auf den Aktivsubstanzgehalt -
(a) 2 bis 70 Gew.-% C₈-C₂₂-Fettsäurealkylester,
(b) 1 bis 40 Gew.-% C₈-C₂₂-Fettalkohole,
(c) 10 bis 40 Gew.-% C₈-C₂₂- Alkoholpolyglycolether,
(h) 1 bis 40 Gew.-% C₈-C₂₂-Fettsäurepartialglyceride und
(i) 0 bis 70 Gew.-% Hilfs- und Zusatzstoffe, wobei diese
(e-1) 0,001 bis 2,5 Gew.-% Chitosane und gegebenenfalls
(e-2) 0 bis 2,5 Gew.-% (Desoxy)Ribonucleinsäuren enthalten,
mit der Maßgabe, daß sich die Mengenangaben zu 100 Gew.-% ergänzen

17. Verwendung nach mindestens einem der Ansprüche 1 bis 16, **dadurch gekennzeichnet, daß** man PIT-Emulsionen einsetzt, enthaltend - bezogen auf den Aktivsubstanzgehalt -
(a) 2 bis 70 Gew.-% C₈-C₂₂-Fettsäurealkylester,
(b) 1 bis 40 Gew.-% C₈-C₂₂-Fettalkohole,
(c) 10 bis 40 Gew.-% C₈-C₂₂-Alkoholpolyglycolether,
(d) 1 bis 40 Gew.-% C₈-C₂₂-Fettsäurepartialglyceride und
(e) 0 bis 70 Gew.-% Hilfs- und Zusatzstoffe, wobei diese
(e-1) 0,001 bis 2,5 Gew.-% (Desoxy)Ribonucleinsäuren und gegebenenfalls
(e-2) 0 bis 2,5 Gew.-% Chitosane enthalten,
mit der Maßgabe, daß sich die Mengenangaben zu 100 Gew.-% ergänzen.

## Claims

1. The use of PIT emulsions containing
(a) C₈₋₂₂ fatty acid alkyl esters,
(b) C₈₋₂₂ fatty alcohols,
(c) C₈₋₂₂ alcohol polyglycol ethers and
(d) C₈₋₂₂ fatty acid partial glycerides as impregnating and softening formulations for papers, nonwovens and cloths.

2. The use claimed in claim 1, **characterized in that** fatty acid alkyl esters corresponding to formula **(I)**:
**R**^{**1**}**CO-OR**^{**2**} (I)
in which R¹CO is a linear or branched, saturated or unsaturated acyl group containing. 8 to 22 carbon atoms and R² is a linear or branched alkyl and/or alkenyl group containing 6 to 22 carbon atoms, are used as component (a).

3. The use claimed in claim 2 or 3, **characterized in that** fatty alcohols corresponding to formula **(II)**:
**R**^{**3**}**OH** (II)
in which R³ is a linear or branched alkyl and/or alkenyl group containing 8 to 22 carbon atoms, are used as component (b).

4. The use claimed in at least one of claims 1 to 4, **characterized in that** alcohol polyglycol ethers corresponding to formula **(III)**:
**R**^{**4**}**O(CH**_{**2**}**CHR**^{**5**}**O)**_{**n**}**H** (III)
in which R⁴ is a linear or branched alkyl and/or alkenyl group containing 8 to 22 carbon atoms, R⁵ is hydrogen or methyl and n is a number of 1 to 50, are used as component (c).

5. The use claimed in at least one of claims 1 to 4, **characterized in that** partial glycerides corresponding to formula **(IV)**:
**HOCH**_{**2**}**CH(OH)CH**_{**2**}**OCOR**^{**6**} (IV)
in which R⁶CO is a linear or branched, saturated or unsaturated acyl group containing 8 to 22 carbon atoms, are used as component (d).

6. The use claimed in at least one claims 1 to 5, **characterized in that** PIT emulsions containing - based on their active substance content -
(a) 1 to 70% by weight of C₈₋₂₂ fatty acid alkyl esters,
(b) 1 to 40% by weight of C₈₋₂₂ fatty alcohols,
(c) 1 to 40% by weight of C₈₋₂₂ alcohol polyglycol ethers,
(d) 1 to 40% by weight of C₈₋₂₂ fatty acid partial glycerides and optionally
(e) 0 to 70% by weight of auxiliaries and additives, with the proviso that the quantities add up to 100% by weight, are used.

7. The use claimed in at least one of claims 1 to 6, **characterized in that** PIT emulsions with an active substance content of 0.5 to 80% by weight are used.

8. The use claimed in at least one of claims 1 to 7, **characterized in that** PIT emulsions containing skin-care oils as auxiliaries and additives are used.

9. The use claimed in at least one of claims 1 to 8, **characterized in that** PIT emulsions containing nonionic, amphoteric and/or cationic co-emulsifiers as auxiliaries and additives are used.

10. The use claimed in at least one of claims 1 to 9, **characterized in that** PIT emulsions containing active substances as auxiliaries and additives are used.

11. The use claimed in at least one of claims 1 to 10, **characterized in that** PIT emulsions containing (e-1) chitosans and/or (e-2) (deoxy)ribonucleic acids as active substances are used.

12. The use claimed in at least one of claims 1 to 11, **characterized in that** chitosans with an average molecular weight of 10,000 to 5,000,000 dalton are used as component (e-1).

13. The use claimed in at least one of claims 1 to 12, **characterized in that** chitosans with an average molecular weight of 30,000 to 100,000 dalton are used as component (e-1).

14. The use claimed in at least one of claims 1 to 13, **characterized in that** chitosans with an average molecular weight of 800,000 to 1,200,000 dalton are used as component (e-1).

15. The use claimed in at least one of claims 1 to 14, **characterized in that** chitosans selected from the group consisting of anionically derivatized chitosans, nonionically derivatized chitosans and cationically derivatized chitosans are used as component (e-1).

16. The use claimed in at least one of claims 1 to 15, **characterized in that** PIT emulsions containing - based on active substance content -
(a) 2 to 70% by weight of C₈₋₂₂ fatty acid alkyl esters,
(b) 1 to 40% by weight of C₈₋₂₂ fatty alcohols,
(c) 10 to 40% by weight of C₈₋₂₂ alcohol polyglycol ethers,
(d) 1 to 40% by weight of C₈₋₂₂ fatty acid partial glycerides and
(e) 0 to 70% by weight of auxiliaries and additives, these auxiliaries and additives containing (e-1) 0.001 to 2.5% by weight of chitosans and optionally (e-2) 0 to 2.5% by weight of (deoxy)ribonucleic acids,
with the proviso that the quantities shown add up to 100% by weight, are used.

17. The use claimed in at least one of claims 1 to 15, **characterized in that** PIT emulsions containing - based on active substance content -
(a) 2 to 70% by weight of C₈₋₂₂ fatty acid alkyl esters,
(b) 1 to 40% by weight of C₈₋₂₂ fatty alcohols,
(c) 10 to 40% by weight of C₈₋₂₂ alcohol polyglycol ethers,
(d) 1 to 40% by weight of C₈₋₂₂ fatty acid partial glycerides and
(e) 0 to 70% by weight of auxiliaries and additives, these auxiliaries and additives containing (e-1) 0.001 to 2.5% by weight of (deoxy)ribonucleic acids and optionally (e-2) 0 to 2.5% by weight of chitosans,
with the proviso that the quantities shown add up to 100% by weight, are used.

## Revendications

1. Utilisation d'émulsions PIT contenant :
a) des alkylesters d'acide gras en C₈ - C_{22,}
b) des alcools gras en C₈ - C_{22,}
c) des polyglycoléthers d'alcool en C₈ - C₂₂, et
d) des glycérides partiels d'acides gras en C₈ - C₂₂, comme agents d'imprégnation et d'avivage pour des papiers, des non-tissés et des tissus.

2. Utilisation selon la revendication 1,
**caractérisée en ce qu'**
on met en oeuvre comme composant (a) des alkylesters d'acide gras de formule (I)
R¹CO - OR² (I)
dans laquelle R¹CO représente un reste acyle linéaire ou ramifié, saturé ou non saturé, ayant de 8 à 22 atomes de carbone et R² représente un reste alkyle et/ou alkényle linéaire ou ramifié ayant de 6 à 22 atomes de carbone.

3. Utilisation selon les revendications 2 et/ou 3,
**caractérisée en ce qu'**
on met en oeuvre comme composant (b) des alcools gras de formule (II)
R³ OH (III)
dans laquelle R³ représente un reste alkyle et/ou alkényle linéaire ou ramifié ayant de 8 à 22 atomes de carbone.

4. Utilisation selon au moins une des revendications 1 à 3,
**caractérisée en ce qu'**
on met en oeuvre comme composant (c) un polyglycoléther d'alcool de formule (III)
R⁴ O (CH₂CHR⁵O) ₙ H (III)
dans laquelle R⁴ représente un reste alkyle et/ou alkényle linéaire ou ramifié ayant de 8 à 22 tomes de carbone, R⁵ représente de l'hydrogène ou un méthyle et n représente des nombres allant de 1 à 50.

5. Utilisation selon au moins une des revendications 1 à 4,
**caractérisée en ce qu'**
on met en oeuvre comme composant (d) des glycérides partielles de formule (IV)
HOCH₂CH(OH)CH₂OCOR⁶ (IV)
dans laquelle R⁶CO représente un reste acyle linéaire ou ramifié, saturé ou non saturé ayant de 8 à 22 atomes de carbone.

6. Utilisation selon au moins une des revendications 1 à 5,
**caractérisée en ce qu'**
on met en oeuvre des émulsions PIT contenant, rapporté à la teneur en substance active,
(a) de 2 à 70 % en poids d'alkylester d'acides gras en C₈ - C₂₂,
(b) de 1 à 40 % en poids d'alcools gras en C₈ - C₂₂,
(c) de 10 à 40 % en poids de polyglycoléther d'alcools en C₈ - C₂₂,
(d) de 1 à 40 % en poids de glycérides partiels d'acides gras en C₈ - C₂₂ et éventuellement
(e) de 0 à 70 % en poids d'adjuvants et d'additifs avec la précision que les données en quantité se complètent à 10 % en poids.

7. Utilisation selon au moins une des revendications 1 à 6,
**caractérisée en ce qu'**
on met en oeuvre des émulsions PIT ayant une teneur en substance active dans la zone de 0,5 à 80 % en poids.

8. Utilisation selon au moins une des revendications 1 à 7,
**caractérisée en ce qu'**
on met en oeuvre des émulsions PIT qui renferment comme substances adjuvantes et d'addition des huiles soignantes.

9. Utilisation selon au moins une des revendications 1 à 8,
**caractérisée en ce qu'**
on met en oeuvre des émulsions PIT qui renferment comme adjuvants et additifs des co-émulsionnants non ioniques, amphotères et/ou cationiques.

10. Utilisation selon au moins une des revendications 1 à 9,
**caractérisée en ce qu'**
on met en oeuvre des émulsions PIT qui renferment des principes actifs comme adjuvants et additifs.

11. Utilisation selon au moins une des revendications 1 à 10,
**caractérisée en ce qu'**
on met en oeuvre des émulsions PIT qui renferment comme principes actifs :
(e-1) de chitosanes et/ou
(e-2) des acides (désoxy)ribonucléiques.

12. Utilisation selon au moins une des revendications 1 à 11,
**caractérisée en ce qu'**
on met en oeuvre comme composant (e-1) des chitosanes ayant un poids moléculaire moyen de 10.000 à 5.000.000 daltons.

13. Utilisation selon au moins une des revendications 1 à 12,
**caractérisée en ce qu'**
on met en oeuvre comme composant (e-1) des chitosanes ayant un poids moléculaire moyen allant de 30.000 à 100.000 daltons.

14. Utilisation selon au moins une des revendications 1 à 13,
**caractérisée en ce qu'**
on met en oeuvre comme composant (e-1) des chitosanes ayant un poids moléculaire moyen de 800.000 à 1.200.000 daltons.

15. Utilisation selon au moins une des revendications 1 à 14,
**caractérisé en ce qu'**
on met en oeuvre comme composant (e-1) des chitosanes choisis dans le groupe qui consiste en des chitosanes dérivés d'une manière anionique, d'une manière non ionique et d'une manière cationique.

16. Utilisation selon au moins une des revendications 1 à 15,
**caractérisée en ce qu'**
on met en oeuvre des émulsions PIT contenant - rapporté à la teneur en substance active -
a) de 2 à 70 % en poids d'alkylester d'acide gras en C₈ - C₂₂,
b) de 1 à 40 % en poids d'alcools gras en C₈ - C₂₂,
c) de 10 à 40 % en poids de polyglycoléther d'alcool en C₈ - C₂₂
d) (h) de 1 à 40 % en poids de glycérides partiels d'acide gras en C₈-C₂₂ et
e) de O à 70 % en poids d'adjuvants et d'additifs parmi lesquels ceux-ci renferment :
(e-1) de 0,001 à 2,5 % en poids de chitosanes, et éventuellement
(e-2) de 0 à 2,5 % en poids d'acides (désoxy)ribonucléique,
avec la précision que les données en poids se complètent à 100 % en poids.

17. Utilisation selon au moins une des revendications 1 à 16,
**caractérisée en ce qu'**
on met en oeuvre des émulsions PIT contenant, rapporté à la teneur en substance active,
(a) de 2 à 70 % en poids d'alkylester d'acides gras en C₈ - C₂₂,
(b) de 1 à 40 % en poids d'alcools gras en C₈ - C₂₂,
(c) de 10 à 40 % en poids de polyglycoléther d'alcool en C₈ - C₂₂,
(d) de 1 à 40 % en poids de glycérides partiels d'acide gras en C₈ - C₂₂, et
(e) de 0 à 70 % en poids d'adjuvants et d'additifs parmi lesquels ceux-ci renferment :
(e-1) de 0,001 à 2,5 % en poids d'acides (désoxy)ribonucléiques, et éventuellement
(e-2) de O à 2,5 % en poids de chitosanes
avec la précision que les données en quantités se complètent à 100 % en poids.
